# EUROPÄISCHE PATENTSCHRIFT

(11) **EP 2 802 550 B1**
(45) Veröffentlichungstag und Bekanntmachung des Hinweises auf die Patenterteilung: **13.01.2016**
(21) Anmeldenummer: 13766563.4
(22) Anmeldetag: 27.09.2013
(51) Int. Cl.: C07C 45/50, C07F 9/6574, B01J 31/02, B01J 31/18, B01J 19/24

(54) **LANGZEITSTABILES VERFAHREN ZUR HERSTELLUNG VON C5-ALDEHYDEN**
LONG-TERM STABLE PROCESS FOR THE PREPARATION OF C5-ALDEHYDES
PROCÉDÉ STABLE À LONG TERME POUR LA PRÉPARATION D'ALDÉHYDES EN C5

(30) Priorität: 12.10.2012 DE 102012218627; 12.10.2012 DE 102012218625; 12.10.2012 DE 102012218629; 12.10.2012 DE 102012218630
(43) Veröffentlichungstag der Anmeldung: 19.11.2014
(73) Patentinhaber: Evonik Degussa GmbH, 45128 Essen (DE)
(72) Erfinder: FRIDAG, Dirk, 45721 Haltern am See (DE); FRANKE, Robert, 45772 Marl (DE); HESS, Dieter, 45770 Marl (DE); SCHWARZ, Markus, 45721 Haltern am See (DE); DYBALLA, Katrin Marie, 45657 Recklinghausen (DE); LUEKEN, Hans-Gerd, 45770 Marl (DE); HAMERS, Bart, 5961 VG Horst (NL); ERNST, Uwe, 45770 Marl (DE)
(86) Internationale Anmeldenummer: PCT/EP2013/070208
(87) Internationale Veröffentlichungsnummer: WO 2014/056732

(56) Entgegenhaltungen:
- EP-B1- 2 280 920

## Beschreibung

Die Erfindung betrifft ein Verfahren zur Herstellung von Aldehyden mit fünf Kohlenstoffatomen, bei welchem ein 10 Gew.-% bis 50 Gew.-% lineare Butene und weniger als 5 Gew.-% 1-Buten enthaltendes Einsatzgemisch mit Synthesegas in Gegenwart eines Rhodium sowie mindestens einen Bisphosphit-Liganden umfassenden Katalysatorsystems hydroformyliert wird, wobei die Hydroformylierung in einem Reaktor erfolgt, aus welchem über einen Betriebszeitraum kontinuierlich ein Kreisgas enthaltend zumindest ein Teil der Produkte sowie nicht umgesetzte Edukte der Hydroformylierung abgezogen, teilkondensiert und nicht kondensierte Anteile des Kreisgases in den Reaktor zurückgeführt wird, und wobei nach Ablauf des Betriebszeitraums die Hydroformylierung unterbrochen, der Reaktor von Reaktionsrückständen befreit und die Hydroformylierung wieder aufgenommen wird.

Zu den Aldehyden mit fünf Kohlenstoffatomen (kurz: C₅-Aldehyde) gehören n-Pentanal (Valeraldehyd), iso-Pentanal (Isovaleradelhyd), sec-Pentanal (2-Methylbutanal) und tert-Pentanal (Pivalaldehyd).

Pentanale dienen als Ausgangsstoffe zur Erzeugung von Pentanolen, Pentansäuren und Pentylaminen. Durch Aldolkondensation und Totalhydrierung des Aldolkondensats können aus ihnen Decanole gewonnen werden, die Zwischenprodukte für die Herstellung von Weichmachern, Detergenzien und Schmiermitteln sind. Durch ihre Aldolkondensation, Hydrierung der olefinischen Doppelbindung des Aldolkondensats und anschließende Oxidation der aldehydischen Gruppe können Decansäuren erhalten werden, die beispielsweise zur Herstellung von Schmiermitteln oder Detergenzien verwendet werden können.

Pentanale können durch Hydroformylierung von ungesättigten Verbindungen mit vier Kohlenstoffatomen erhalten werden. Unter Hydroformylierung (Oxo-Reaktion) versteht man allgemein die Umsetzung von ungesättigten Verbindungen wie insbesondere Olefinen (Alkene) mit Synthesegas (Wasserstoff und Kohlenmonoxid) in Aldehyde, deren Anzahl an Kohlenstoffatome um 1 höher liegt als die Anzahl der Kohlenstoffatome der Ausgangsverbindungen. Zur Herstellung von C₅-Aldehyden werden dementsprechend C₄-Olefine hydroformyliert.

Eine gute Übersicht über den Stand der Hydroformylierung von Olefinen findet sich in
B. Cornils, W. A. Herrmann, "Applied Homogeneous Catalysis with Organometallic Compounds", Vol. 1 & 2, VCH, Weinheim, New York, 1996
sowie in
R. Franke, D. Selent, A. Börner, "Applied Hydroformylation", Chem. Rev., 2012, DOI:10.1021/cr3001803.

In der industriellen Praxis werden die C₄-Olefine aber nicht als hochpreisige Reinstoffe eingesetzt, sondern als rohe Gemische enthaltend unterschiedliche isomere C₄-Olefine. Da die einzelnen C₄-Olefine in der Hydroformylierung unterschiedliche C₅-Aldehyde bilden, entsteht bei der Hydroformylierung dieser C₄-Gemische auch ein Gemisch unterschiedlicher C₅-Aldehyde.

Konkret werden bei der industriellen Pentanalherstellung C₄-Kohlenwasserstoffgemische verwendet, die 1-Buten, trans-2-Buten, cis-2-Buten und Isobuten enthalten. Neben den genannten C₄-Olefinen können auch Olefine mit mehr oder weniger Kohlenstoffatome oder Alkane (Paraffine) im Einsatzgemisch enthalten sein.

Entsprechend der Stellung der C-C-Doppelbindung in den ungesättigten C₄-Verbindungen und in Abhängigkeit der Reaktionsbedingungen liefert deren Hydroformylierung lineare und verzweigte C₅-Aldehyde bzw. C₅-Aldehydgemische in unterschiedlicher Selektivität.

Sollen aus dem C₅-Aldehydgemisch später Weichmacher oder Detergenzien hergestellt werden, so ist es wichtig, dass das Pentanalgemisch möglichst ausschließlich aus der linearen Verbindung n-Pentanal (Valeraldehyd) besteht, bzw. dass der Anteil an verzweigten C₅-Aldehyden, wie insbesondere 2-Methylbutanal, möglichst gering ist. Mithin gilt es, die Hydroformylierung in Richtung ihres begehrtesten Reaktionsprodukts - dem Valeraldehyd -zu optimieren.

1-Buten kann in über 90%-iger Selektivität zu n-Pentanal hydroformyliert werden. Als Katalysatoren werden dazu meistens Komplexe aus Rhodium und Monophosphinen verwendet. Ein Standardkatalysator ist beispielsweise ein Komplex, bestehend aus Rhodium und Triphenylphosphin. Die Umsetzung kann in homogener Phase, wie etwa in EP0562451 beschrieben, oder in heterogener Phase, wie etwa in DE02627354 beschrieben, durchgeführt werden.

Die selektive Herstellung von n-Pentanal aus 2-Butenen oder von Gemischen davon ist wesentlich schwieriger. In DE10108474, DE10108475, DE10108476 und DE10225282 wird die Herstellung von C₅-Aldehydgemischen durch Hydroformylierung eines Gemisches linearer Butene beschrieben. Die technischen Lehren aller dieser Schriften haben gemeinsam, dass in mindestens einem Hydroformylierungsschritt ein Rhodiumkatalysator mit einem Diphosphinliganden, der ein Xanthengerüst aufweist, verwendet wird. Mit diesem Katalysator können 2-Butene unter isomerisierenden Bedingungen hydroformyliert werden. Das Verhältnis von n-Pentanal zu 2-Methylbutanal liegt bei bestenfalls 85 zu 15. Die Schriften DE10108474 und DE10108475 beschreiben Verfahren, bei denen die Hydroformylierung zweistufig erfolgt. In der ersten Hydroformylierungsstufe wird unter Verwendung eines Katalysators, bestehend aus Rhodium und einem Monophosphin als Ligand, 1-Buten in einer Selektivität von 90 % zu n-Pentanal umgesetzt. Die nicht umgesetzten Butene, hauptsächlich 2-Butene, werden in der zweiten Hydroformylierungsstufe unter Verwendung des oben genannten Rhodium/Bisphosphins umgesetzt. Die Schriften DE10108476 und DE10225282 beschreiben einstufige Hydroformylierungsverfahren.

Höhere Selektivitäten an n-Pentanal bei der Hydroformylierung von 2-Butenen können bei Verwendung eines Katalysators, bestehend aus Rhodium und sperrigen aromatischen Bisphosphiten erhalten werden, wie sie beispielsweise in EP0213639 beschrieben werden. Allerdings nimmt die Selektivität mit der Zeit stark ab.

In DE102005042464 werden für die Hydroformylierung von Olefinen Katalysatorsysteme genannt, die einen Komplex, bestehend aus Rhodium und einer phosphororganischen Verbindung sowie ein sterisch gehindertes sekundäres Amin enthalten. Diese Katalysatorsysteme zeichnen sich durch hohe Langzeitstabilität aus. Sie können für die Hydroformylierung von Olefinen mit drei bis sechszehn Kohlenstoffatomen eingesetzt werden. In den Beispielen wurde lediglich 1-Octen hydroformyliert. Es entstanden Gemische mehrerer C₉-Aldehyde, über deren Isomerenverteilung aber nichts ausgesagt ist.

Neben der Wahl des Katalysatorsystems haben der apparative Aufbau der Hydroformylierungsanlage, kurz Oxo-Anlage, und ihre Betriebsweise signifikanten Einfluss auf die Wirtschaftlichkeit des Verfahrens.

Eine Möglichkeit zur technischen Umsetzung von Hydroformylierungen im großindustriellen Maßstab ist der Kreisgasprozess. Bei einem Kreisgasprozess (engl.: gas recyle; stripping reactor process) werden die Produkte der Hydroformylierung gasförmig mit überschüssigen Synthesegas aus dem Reaktor ausgetragen. Eine allgemeine Schilderung der Hydroformylierung im Kreisgasverfahren findet sich in:
Van Leeuwen, Piet W.N.M und Claver, Carmen (Edit.): Rhodium Catalyzed Hydroformylation. Catalysis by Metal Complexes. Volume 22. Kluwer, 2000, Seiten 212f..

Der Vorteil einer nach dem Kreisgas-Verfahren arbeitende Oxo-Anlage ist ihr einfacher apparativer Aufbau. Aufgrund der vergleichsweise geringen Flüchtigkeit der C₅-Aldehyde und insbesondere der in Nebenreaktionen gebildeten Hochsieder gelten Kreisgasverfahren für die Pentanal-Herstellung allerdings als inakzeptabel; vgl. van Leeuwen, a.a.O.

Nichtsdestotrotz hat es Versuche gegeben, C₅-Aldehyde aus C₄-Olefingemischen im Kreisgasverfahren herzustellen:
So beschreibt EP0016285B2 ein Kreisgasverfahren zur Herstellung von Valeraldehyd aus einem Gemisch enthaltend 2.23 % n-Butan, 1.06 % iso-Butan, 69.88 % 1-Buten, 10.06 % cis-2-Buten und 15.1 % trans-2-Buten. Da offenbar aufgrund des hohen Anteils an 1-Buten im Ausgangsgemisch keine hohen Anforderungen an die Selektivität des Katalysatorsystems gestellt werden, wird ein vergleichsweise simpler Triorganophosphin-Ligand im Katalysatorkomplex eingesetzt. Auf diese Weise kann Valeraldehyd mit einer einfachen Kreisgas-Anlage im industriellen Maßstab produziert werden. Bei der wirtschaftlichen Betrachtung dieses Verfahrens ist allerdings zu beachten, dass 1-Buten ein begehrtes Co-Monomer für die Herstellung hochwertiger Kunststoffe ist. Somit steht Valeraldehyd in Rohstoffkonkurrenz mit anderen auf 1-Buten basierten Produkten. Wirtschaftlich wünschenswert ist deswegen die Herstellung von C₅-Aldehydgemischen, die reich an n-Pentanal sind, aus C₄-Olefingemischen, die arm an 1-Buten sind.

Ein entsprechendes Verfahren der eingangs genannten Gattung ist aus EP2280920B1 bekannt. Die Erfinder gehen von dieser Schrift als nächstliegenden Stand der Technik aus.

Bei der in EP2280920B1 praktizierten Kreisgas-Hydroformylierung zur Herstellung von Valeraldehyd wird ein Einsatzgemisch verwendet, welches 35 % 2-Butene enthält und lediglich 1 % 1-Buten. Der Rest ist inertes Butan. Das extrem an 1-Buten arme Gemisch wird mit Hilfe eines symmetrischen Bisphosphit-Liganden hydroformyliert, welcher durch Zugabe eines sterisch gehinderten, sekundären Amins stabilisiert wird. Als Lösungsmittel wird Isononylbenzoat erwähnt.

Mit diesem Katalysatorsystem werden Butenumsätze von 60 bis 75 % erzielt. Die prozentuale Verteilung zwischen n-Pentanal und 2-Methylbutanal, bzw. die n/iso-Selektivität, betrug 95 % zu 5 %.

Nachteil dieses Katalysatorsystems ist, dass es nach einer Betriebsdauer von etwa 1000 h einen Niederschlag an der Wand des Kreisgas-Reaktors verursacht. Eine Analyse des Niederschlags ergab, dass es sich dabei um phosphorhaltige Folgeprodukte des Bisphosphit-Liganden und des eingesetzten Amins handelt.

Dies bedeutet, dass der in EP2280920B1 beschriebene Ligand sich trotz des als Stabilisators eingesetzten Amins bereits nach einer für ein industriell praktizierbares Verfahren relativ kurzen Betriebsdauer abbaut, sodass der Umsatz der Reaktion sinkt.

In US 5364950, wie auch in US 5763677 und in *"*Catalyst Separation, Recovery and Recycling", herausgegeben v. D.J. Cole-Hamilton, R.P. Tooze, 2006, NL, Seiten 25-26, wird die Bildung von sogenannten *"Poisoning Phosphites"* als Neben- bzw. Ligandenabbaureaktion beschrieben. *Diese "Poisoning Phosphites"* bilden sich bei der Verwendung von arylphosphit-modifizierten Rhodium-Komplexen während der Hydroformylierungsreaktion. Hierbei kommt es im Zuge des Ligandenabbaus zu einem Austausch einer Arylgruppe durch eine Alkylgruppe des Hydroformylierungsproduktes.

Neben der Bildung der unerwünschten *"Poisoning Phosphites"* kann der Phosphitligand auch im Zuge einer Hydrolysereaktion durch die bei der Aldehydkondensation gebildeten Wasserspuren abgebaut werden. Eine Konsequenz aus diesen Abbaureaktionen der Liganden ist, dass die Konzentration an hydroformylierungsaktiven Rhodiumkomplexspezies im Laufe der Zeit abnimmt.

Um dem zu begegnen, muss kontinuierlich Frischkatalysator hinzugeführt werden, was natürlich mit zusätzlichen Katalysatorkosten verbunden ist. Darüber hinaus führt der stetige Abbau des Liganden allmählich zu einer Versumpfung des Reaktors, sodass kaum noch Kreisgas in den Reaktor eingeblasen werden kann. Mithin muss die Hydroformylierung unterbrochen, der Reaktor von Reaktionsrückständen wie insbesondere dem Niederschlag befreit, mit Frischkatalysator beschickt und die Hydroformylierung wieder aufgenommen werden. Aus wirtschaftlicher Sicht ist das tatsächlich inakzeptabel, sofern die Betriebsdauer des Prozesses lediglich 1000 h beträgt, bis der Niederschlag eine Reinigung und Frischbeschickung des Reaktors erforderlich macht.

Zur Lösung dieses Problems schlägt EP2280920B1 eine kontinuierliche Filterung vor, um unlösliche Folgeprodukte des Bisphosphit-Liganden aus dem Reaktionssystem zu entfernen. Diese Maßnahme verlängert zwar die Betriebsdauer, macht aber die kostenintensive Zugabe von Frischkatalysator nicht entbehrlich. Darüber hinaus erfordert der Filterkreislauf zusätzlichen apparativen Aufwand.

Nach alldem konnte bisher kein wirtschaftlich überzeugendes Konzept zur Herstellung von C₅-Aldehydgemischen mit großem Valeraldehyd-Anteil aus C₄-Olefingemischen mit geringem 1-Buten-Anteil angegeben werden.

Entsprechend liegt der Erfindung die Aufgabe zu Grunde, das aus EP2280920B1 bekannte Verfahren wirtschaftlicher zu gestalten.

Gelöst wird die Aufgabe dadurch, dass als Ligand ein Bisphosphit der Formel (1) und/oder (2) eingesetzt wird, 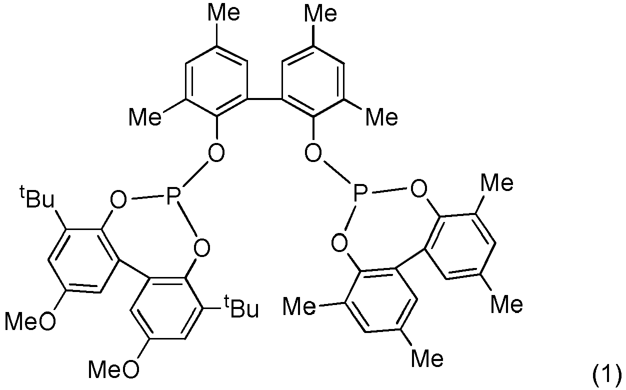 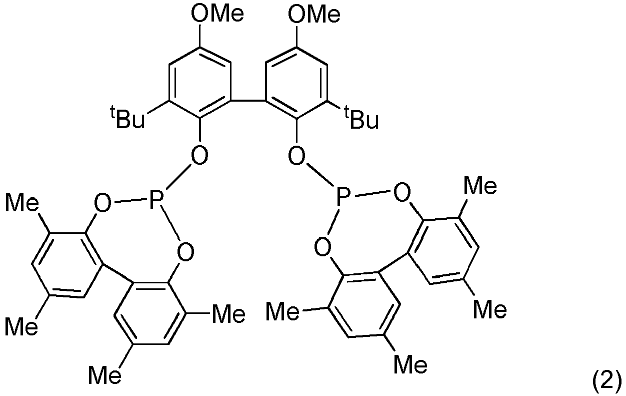 dass der Betriebszeitraum auf über 8000 h ausgedehnt wird, und dass während des andauernden Betriebszeitraums auf eine Abscheidung fester Reaktionsrückstände aus dem Reaktor verzichtet wird.

Gegenstand der Erfindung ist mithin ein Verfahren zur Herstellung von Aldehyden mit fünf Kohlenstoffatomen, bei welchem ein 10 Gew.-% bis 50 Gew.-% lineare Butene und weniger als 5 Gew.-% 1-Buten enthaltendes Einsatzgemisch mit Synthesegas in Gegenwart eines Rhodium sowie mindestens einen Bisphosphit-Liganden der Formel (1) und/oder der Formel (2) umfassenden Katalysatorsystems hydroformyliert wird, wobei die Hydroformylierung in einem Reaktor erfolgt, aus welchem über einen mindestens 8000 h andauernden Betriebszeitraum kontinuierlich ein Kreisgas enthaltend zumindest ein Teil der Produkte sowie nicht umgesetzte Edukte der Hydroformylierung abgezogen, teilkondensiert und nicht kondensierte Anteile des Kreisgases in den Reaktor zurückgeführt wird, wobei nach Ablauf des Betriebszeitraums die Hydroformylierung unterbrochen, der Reaktor von Reaktionsrückständen befreit und die Hydroformylierung wieder aufgenommen wird, und wobei während des andauernden Betriebszeitraums auf eine Abscheidung fester Reaktionsrückstände aus dem Reaktor verzichtet wird.

Die Erfindung beruht auf der Erkenntnis, dass der in den Formeln (1) und (2) wiedergegebene Ligand in dem betrachteten Reaktionssystem selbst über einen langen Betriebszeitraum von über 8000 h nur geringfügig abgebaut wird, weswegen die Betriebsintervalle zwischen der Reinigung und der Frischbeschickung des Reaktors signifikant verlängert werden können, ohne dabei auf eine kontinuierliche Abscheidung fester Reaktionsrückstände angewiesen zu sein. Überraschenderweise erzielt das erfindungsgemäße Katalysatorsystem noch dazu eine n/iso-Selektivität, die eine Herstellung großer Mengen n-Valeraldehyd aus Einsatzströmen ermöglicht, die deutlich mehr 2-Butene enthalten als 1-Buten. Die Wirtschaftlichkeit des erfindungsgemäßen Verfahrens ist damit wesentlich höher als die des in EP2280920B1 geschilderten Prozesses.

Das erfindungsgemäß eingesetzte, Rh-basierte Katalysatorsystem weist den Bisphosphit-Liganden der Formel (1) und/oder den Bisphosphit-Liganden der Formel (2) auf. Dies bedeutet, dass in dem Katalysatorsystem entweder der unsymmetrische Ligand der Formel (1) oder der symmetrische Ligand der Formel (2) oder beide Liganden enthalten sind. Besonders bevorzugt enthält das Katalysatorsystem beide Liganden. Weitere Liganden braucht das Katalysatorsystem nicht enthalten. Deshalb stellen die Liganden der Formel (1) und (2) bevorzugt die einzigen Liganden dar, die im Reaktionssystem gegenwärtig sind.

Im Übrigen wird darauf hingewiesen, dass im Reaktionssystem auch freie Liganden vorkommen können, also Bisphosphite, die nicht mit an einem Rhodium-Kern koordiniert sind. Diese freien Liganden sind nicht katalytisch aktiv und zählen deswegen im Sinne der Erfindung nicht zum Katalysatorsystem.

Wie bereits erwähnt wird vorzugsweise ein Gemisch der Bisphosphit-Liganden der Formel (1) und der Formel (2) eingesetzt. Allerdings hat sich gezeigt, dass der unsymmetrische Bisphosphit-Ligand der Formel (1) katalytisch aktiver, selektiver und stabiler ist als sein symmetrisches Derivat nach der Formel (2). Aus diesem Grunde sollte das molare Verhältnis der Bisphophit-Liganden der Formel (1) zu dem der Bisphosphit-Liganden der Formel (2) zwischen 10 und 20 liegen. Dieses Verhältnis bezieht sich auf die gesamte Hydroformylierung. Dies bedeutet, dass im Reaktionsgemisch die 10- bis 30-fache Menge an Ligand (1) vorhanden ist bezogen auf den Gesamtgehalt Ligand (2). Bei der Berechnung dieses Verhältnisses geht nicht nur die an Rhodium koordinierte, katalytisch aktive Spezies ein, sondern auch die nicht koordinierten, freien Liganden.

Das molare Verhältnis des Rhodiums zu der Summe der beiden Bisphosphit-Liganden (1) und (2) - das sogenannte Ligand/Rhodium-Verhältnis - liegt vorzugsweise in einem Bereich von 1 bis 100. Dies bedeutet, dass auf jeden Rhodium-Kern ein 1 bis 100 Bisphosphit-Liganden gezählt werden. Diese Werte gelten wieder für das gesamte Reaktionsgemisch. Das Ligand/Rhodium-Verhältnis liegt insbesondere im Bereich von 1 bis 20, besonders bevorzugt liegt es im Bereich von 1 bis 2.

Die Konzentration des Rhodiums im Reaktionsgemisch liegt in einem Bereich von 1 bis 1000 Massen-ppm, insbesondere in einem Bereich von 20 bis 300 Massen-ppm und ganz besonders im Bereich von 40 bis 150 Massen-ppm.

Das Katalysatorsystem kann nicht als einsatzbereiter, aktiver Komplex in den Prozess eingebracht werden sondern muss in situ, also im Reaktor herstellt werden.

Hierfür wird der aktive Komplex innerhalb des Hydroformylierungsreaktors unter Hydroformylierungsbedingungen in Gegenwart der Bisphosphit-Liganden (1) und/oder (2) aus stabilen, leichtlagerbaren Rhodiumverbindungen hergestellt. Geeignete Rhodiumverbindungen dafür sind zum Beispiel Rhodium(II)- und Rhodium(III)-salze, wie Rhodium(III)chlorid, Rhodium(III)nitrat, Rhodium(III)sulfat, Kalium-Rhodiumsulfat, Rhodium(II)- bzw. Rhodium(III)-carboxylat, Rhodium(II)- und Rhodium(III)acetat, Rhodium(II)octanoat, Rhodium(II)nonanoat, Rhodium(III)oxid, Salze der Rhodium(III)säure, Trisammoniumhexachlororhodat(III). Weiterhin eignen sich Rhodiumkomplexe, wie Rhodiumbiscarbonylacetylacetonat, Acetylacetonatobisethylenrhodium(I). Besonders geeignet sind Rhodiumacetat, Rhodiumoctanoat und Rhodiumnonanoat.

In einer bevorzugten Weiterbildung der Erfindung wird die Hydroformylierung in Gegenwart eines organischen Amins der Formel (3) durchgeführt.

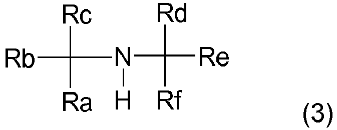

In Formel (3) stellen Ra, Rb, Rc, Rd, Re und Rf gleiche oder unterschiedliche Kohlenwasserstoffreste dar, die auch untereinander verbunden sein können.

Das organische Amin verhindert nämlich die Hydrolyse der eingesetzten Bisphosphite und wirkt somit als Stabilisator. Das molare Verhältnis von der Gesamtheit der Bisphophite zu dem Amin der Formel (3) liegt im Bereich von 0.1 zu 10 bis 10 zu 1, insbesondere in einem Bereich von 5 zu 10 bis 10 zu 5 und ganz besonders in einem Bereich von 0.8 zu 1 bis 1 zu 0.8.

Besonders bevorzugt weist das organische Amin zumindest eine 2,2,6,6-Tetramethylpiperidineinheit auf. Ganz besonders bevorzugt handelt es sich bei dem organischen Amin um ein Sebacinsäuredi-4-(2,2,6,6-tetramethylpiperidinyl)-ester. Letzterer ist unter dem Markennamen Tinuvin® bei der BASF SE erhältlich.

Neben den Edukten, dem Katalysatorsystem und den Produkten kann das Reaktionsgemisch auch ein zusätzliches Lösungsmittel zum Lösen des Katalysatorsystems enthalten. Innerhalb des gesamten Reaktionsgemisches kann das Lösungsmittel 60 bis 80 Gew.-% einnehmen.

Als Lösungsmittel wird vorzugsweise Isononylbenzoat verwendet. Isononylbenzoat (INB) ist der Ester der Benzoesäure mit Isononanol. Es ist bei der Evonik Industries AG unter dem Markennamen Vestinol® INB erhältlich. Es wird unter den CAS-Nummern 27458-94-2, 68515-81-1 oder auch 3452-97-9 geführt. Die Herstellung erfolgt in der Regel durch Veresterung von Isononanol mit Benzoesäure wie in DE10217186 beschrieben. Die Veresterungs- und Behandlungsbedingungen können dabei auch variiert werden, wie beispielsweise in US6635775 für andere Benzoesäureester beschrieben.

Der Vorteil von der Verwendung von INB als Lösungsmittel in der Hydroformylierung besteht darin, dass es eine geringere Ökotoxizität gegenüber den traditionell eingesetzten aromatischen Lösungsmitteln aufweist.

Die Hydroformylierung selbst wird bei üblichen Reaktionsbedingungen durchgeführt, also bei einem Druck zwischen 1 und 20 MPa und bei einer Temperatur zwischen 70°C und 150 °C.

Das Kreisgas sollte bei einer Kondensationstemperatur zwischen 50 °C und 90 °C teilkondensiert werden. Bevorzugt ist ein Bereich zwischen 65 °C und 75 °C, ganz besonders bevorzugt wird das Kreisgas bei 70 °C teilkondensiert. Die Kondensation des Kreisgases bei dieser Temperatur ist deshalb vorteilhaft, weil auf diese Weise ein Großteil der nicht umgesetzten Butene und inertes Butan nicht kondensiert wird und der Reaktion über das Kreisgas wieder zugeführt werden kann. Die Reaktionsprodukte werden aber zum größten Teil auskondensiert und führen somit nicht zu einer ungewollten Folgereaktion im Reaktor.

Erfindungsgemäß beträgt der Betriebszeitraum, über welchen die Hydroformylierung kontinuierlich betrieben wird, bevor sie unterbrochen und der Reaktor von Reaktionsrückständen befreit wird, 8000 Stunden. Das entspricht etwa einem Jahr Dauerbetrieb. Das erfindungsgemäß eingesetzte Katalysatorsystem zeigt jedoch eine solch hohe Langzeitstabilität, dass der Betriebszeitraum sogar mehr als 12000 Stunden betragen kann. Erst dann ist es erforderlich die Hydroformylierung zu unterbrechen und den Reaktor von Reaktionsrückständen zu befreien. Zu den Reaktionsrückständen gehören nicht nur das Katalysatorsystem und dessen feste Abbauprodukte, sondern auch etwaige flüssige Nebenprodukte wie Hochsieder, die aufgrund ihrer Flüchtigkeit nicht kontinuierlich mit dem Kreisgas aus dem Prozess ausgetragen werden.

Hochsieder ist in diesem Zusammenhang eine Sammelbezeichnung für alle Komponenten des Reaktionsgemisches, die einen höheren Siedepunkt als das Lösungsmittel aufweisen. Das erfindungsgemäße Katalysatorsystem bildet in so geringem Maße unerwünschte Hochsieder, dass über eine gesonderte Apparatur zur kontinuierlichen Hochsiederausschleusung sogar verzichtet werden kann. Einfachstenfalls verbleiben die Hochsieder bis zur turnusgemäßen Betriebsunterbrechung im Reaktor und werden dann während der Abschaltung zusammen mit den Katalysatorresten entfernt. Aufgrund seiner geringen Hochsiederbildung macht das erfindungsgemäße Katalysatorsystem die Hydroformylierung von C₄-Olefinen im Kreisgasverfahren erst attraktiv, da mit dem Kreisgas schwer siedende Nebenprodukte kaum ausgetragen werden können.

Im Zuge der Betriebsunterbrechung wird der Reaktor drucklos geschaltet, komplett entleert und gereinigt. Danach wird die Hydroformylierung durch Einbringen der Edukte und eines frischen Katalysatorsystems wieder aufgenommen.

Der darauf folgende Betriebszeitraum kann dann wieder 8000 bzw. 12000 Stunden oder mehr betragen.

Ein weiterer Vorteil des erfindungsgemäßen Katalysatorsystems besteht auch darin, dass es eine besonders hohe n/iso-Regioselektivität aufweist und deswegen in der Lage ist Einsatzgemische zu verarbeiten, die einen geringen Anteil an 1-Buten und deswegen als für die Hydroformylierung maßgebliches Substrat überwiegend die beiden anderen linearen Butene cis-2-Buten und trans-2-Buten enthält. Vorzugsweise weist das Einsatzgemisch 10 bis 50 Gew.-% lineare Butene, weniger als 2 Gew.-% 1-Buten und mindestens 50 Gew.-% inerte Butane auf. Alle angegebenen Gewichtsanteile verstehen sich als auf das gesamte Einsatzgemisch bezogen. Auch der gesondert angegebene Anteil an 1-Buten bezieht sich auf das gesamte Einsatzgemisch und nicht bloß auf die Fraktion der darin enthaltenen linearen Butene.

Das eingesetzte Synthesegas weist vorzugsweise ein molares Verhältnis von Wasserstoff zu Kohlenmonoxid in einem Bereich von 2 zu 1 bis 1 zu 2, insbesondere in einem Bereich von 1.1 zu 0.9 bis 0.9 zu 1.1 auf.

Gegenstand der Erfindung ist auch eine Anlage zur Durchführung des erfindungsgemäßen Verfahrens umfassend einen Reaktor zur Herstellung von Aldehyden mit fünf Kohlenstoffatomen durch Hydroformylierung von linearen Butenen und Synthesegas in Gegenwart eines Rhodium sowie mindestens einen Bisphosphit-Liganden umfassenden Katalysatorsystems, weiter umfassend Mittel zum Abzug eines Kreisgases enthaltend zumindest ein Teil der Produkte sowie nicht umgesetzte Edukte der Hydroformylierung aus dem Reaktor, weiter umfassend Mittel zum Teilkondensieren des Kreisgases und zum Rückführen der nicht kondensierten Anteile des Kreisgases in den Reaktor. Die Anlage zeichnet sich erfindungsgemäß dadurch aus, dass sie in ihrem Reaktor mindestens einen Bisphosphit-Ligand der Formel (1) und/oder (2) enthält.

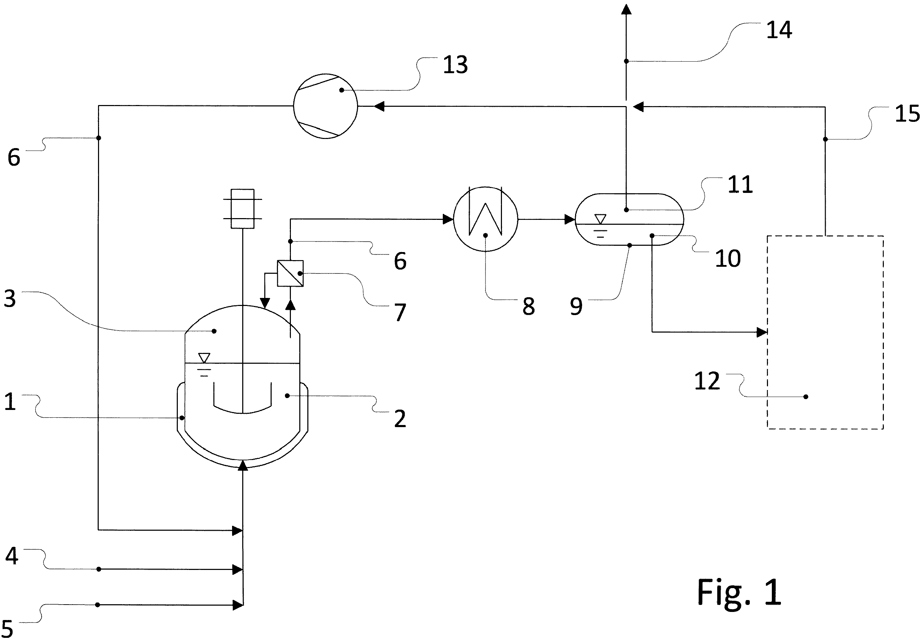

Eine solche Oxo-Anlage zur Durchführung des erfindungsgemäßen Verfahrens ist schematisch in Figur 1 dargestellt.
- Figur 1:: Oxo-Anlage zur Durchführung des Verfahrens (schematisch).

Herzstück der Anlage bildet ein Reaktor 1. Als Reaktor 1 kann jedwede im Stand der Technik bekannte Reaktorbauart genutzt werden, die gas/flüssig-Reaktionen durchzuführen vermag. Besonders bevorzugt kann eine Blasensäule oder ein Rührkessel eingesetzt werden. In der Zeichnung ist ein Rührkesselreaktor dargestellt. Geeignete Blasensäulenreaktoren sind bei Ullmann beschrieben:
Deen, N.G., Mudde, R.F., Kuipers, J.A.M., Zehner, P. and Kraume, M.: Bubble Columns. Ullmann's Encyclopedia of Industrial Chemistry. Published Online: 15 January 2010. DOI: 10.1002/14356007.b04_275.pub2

Unter dem "Reaktor" kann in diesem Zusammenhang auch eine Vielzahl von seriellen oder parallel verschalteten Reaktorgefäßen verstanden werden.

Die Hydroformylierung ist eine zweiphasige Reaktion umfassend ein flüssige Phase 2 und eine gasförmige Phase 3. Die einzelnen Komponenten des Reaktionsgemisches sind entsprechend ihrer Löslichkeit und ihres Partialdrucks auf die flüssige Phase 2 und auf die gasförmige Phase 3 verteilt. Einzig das Katalysatorsystem ist ausschließlich in der flüssigen Phase 2 gelöst. Aufgrund der vollständigen Lösung des Katalysatorsystems innerhalb der flüssigen Phase 2 spricht man auch von einer homogen katalysierten Reaktion.

Während des Betriebszeitraums werden kontinuierlich die Edukte in den Reaktor 1 eingefahren. Als Edukte dienen die Olefine, die in einem Einsatzgemisch 4 enthalten sind und in den Reaktor 1 eingefahren werden. Allerdings stellen die Olefine einen Minderanteil des Einsatzgemisches 4 dar, da das Einsatzgemisch 4 über 50 % aus Butan besteht. Butane verhalten sich innerhalb der Hydroformylierung inert und werden durch den Prozess unverändert durchgeschleust. Hydroformyliert werden ausschließlich die im Einsatzgemisch 4 enthaltenen Olefine.

Das zweite Edukt ist Synthesegas 5, ein Gemisch aus Kohlenmonoxid und Wasserstoff in etwa gleichen molaren Mengen.

Einsatzgemisch 4 und Synthesegas 5 werden gasförmig von unten in den Reaktor 1 eingeblasen, steigen durch die flüssige Phase 2 auf und reagieren dabei im Wesentlichen zu Valeraldehyd. Dazu kommt es zu Nebenreaktionen, die unerwünschte Nebenprodukte wie Isovaleradelhyd, 2-Methylbutanal und Pivalaldehyd sowie Hochsieder mit mehr als fünf Kohlenstoffatomen bilden. Der Anteil der Nebenproduktbildung ist jedoch auf Grund der hohen Selektivität des eingesetzten Katalysatorsystems gering. Gebildeter Aldehyd reagiert auch teilweise mit überschüssigem Wasserstoff weiter zu Alkohol, allerdings auch hier nur in geringem Umfang.

Die C₅-Produkte und nicht umgesetzten Edukte sammeln sich in der Gasphase 3 innerhalb des Reaktors 1 an und werden von dort als Kreisgas 6 abgezogen. Ein Aerosolbrecher 7 verhindert, dass Tröpfchen der flüssigen Phase 2 mit dem Kreisgas 6 mitgerissen werden. Die zurückgehaltenen Tröpfchen fallen zurück in den Reaktor 1.

Das Kreisgas 6 wird in einem Kondensator 8 auf eine Temperatur von etwa 70 °C heruntergekühlt, sodass es teilweise kondensiert. In einem Phasentrennbehältnis 9 wird das Kondensat 10 von dem nicht kondensierten Anteil 11 des Kreisgases getrennt.

Das Kondensat 10 enthält dann im Wesentlichen die Zielprodukte des Prozesses, die C₅-Aldehyde, unerwünschte Nebenprodukte wie Hochsieder sind kaum vorhanden. Das Kondensat 10 wird dann einer hier nicht näher dargestellten Aufarbeitung 12 zugeführt, in welcher das Kondensat 10 in seine Bestandteile destillativ aufgetrennt wird. Eine umfangreiche Beschreibung der Aufarbeitung von C₅-Aldehydgemischen findet sich in EP2280920B1, sowie in DE102009027406A1. An die Aufarbeitung 12 kann sich eine Aldolkondensation anschließen; vgl. DE102009001594A1 und DE102009045139A1.

Die nichtkondensierten Anteile des Kreisgases 11 - das ist im Wesentlichen überschüssiges Synthesegas und C₄-Kohlenwasserstoffe - werden von einem Kreisgasverdichter 13 wieder auf Reaktionsdruck gebracht und zusammen mit frischem Einsatzgemisch 4 und Synthesegas 5 in den Reaktor 1 zurückgeführt.

Die nichtkondensierten Anteile 11 des Kreisgases müssen nicht vollständig zurückgeführt werden. Sinnvoll ist, einen Teil davon als Offgas 14 aus dem Prozess auszuschleusen. Grund dafür ist, dass die nicht kondensierten Anteile 11 des Kreisgases neben Synthesegas und nicht umgesetzten Olefinen auch inerte Alkane wie das im Einsatzgemisch 4 enthaltene Butan enthält. Das inerte Butan wird in der Reaktion nicht verbraucht und vergrößert den Leistungsbedarf des Kreisgasverdichters 13. Deswegen macht es Sinn, einen Teil der nichtkondensierte Anteile 11 als Offgas 14 aus dem Prozess auszuschleusen. Das Offgas 14 wird entweder thermisch oder stofflich verwertet, beispielsweise durch Verbrennen oder durch Rückführen in eine Synthesegasanlage oder einen Cracker.

Gegebenenfalls wird dem rückgeführten Kreisgas noch ein Rezyklat 15 aus der Aldehyd-Aufarbeitung 12 beigemischt. Dabei kann es sich beispielsweise um Synthesegas handeln, welches im Kondensat gelöst vorlag und erst im Zuge der thermischen Aufarbeitung 12 ausgegast ist.

Erfindungsgemäß enthält der Reaktor 1 in der flüssigen Phase 2 gelöst ein Katalysatorsystem umfassend Bisphosphit-Liganden der Formel (1) und/oder (2); dargestellt in der Ausschnittsvergrößerung. Die Liganden (1) und (2) koordinieren sich an einem Rhodium-Kern zur Bildung der aktiven Spezies .Aufgrund der hohen Stabilität dieser Liganden kommt die erfindungsgemäße Oxo-Anlage ohne eine kontinuierliche Filterung aus, vermittels welcher im laufenden Betrieb etwaige Abbauprodukte der Bisphosphit-Liganden aus dem Reaktionssystems abzuscheiden wären. Die erfindungsgemäße Oxo-Anlage weist daher einen apparativ einfachen und zuverlässigen Aufbau auf.

### Bezugszeichenliste

- 1: Reaktor
- 2: flüssige Phase
- 3: gasförmige Phase
- 4: Einsatzgemisch
- 5: Synthesegas
- 6: Kreisgas
- 7: Aerosolbrecher
- 8: Kondensator
- 9: Phasentrenngefäß
- 10: Kondensat
- 11: nicht kondensierte Anteile des Kreisgases
- 12: Aufarbeitung
- 13: Kreisgasverdichter
- 14: Offgas
- 15: Rezyklat

### Beispiele

### Beispiel 1: Hydroformylierung mit dem nicht erfindungsgemäßen Liganden (4) über 1200 h

Der aus EP2280920B1 bekannte, nicht erfindungsgemäße Ligand der Formel (4) wurde in der Hydroformylierung einer Buten/Butan-Mischung eingesetzt.

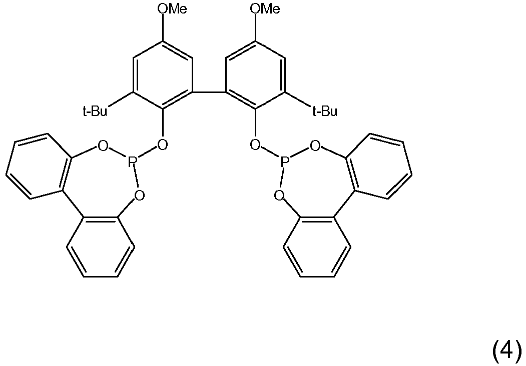

Dabei wurde Ligand (4) mit dem Amin der Formel (5) stabilisiert.

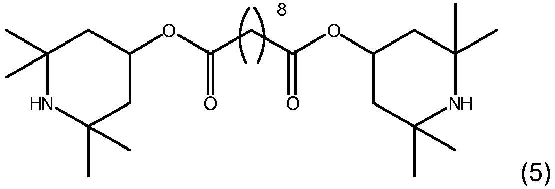

Die kontinuierlich betriebene Versuchsanlage bestand im Wesentlichen aus einem 20 Liter fassenden Druckreaktor mit einem nachgeschalteten Kondensator und Phasentrennbehälter (Gas/Flüssigkeit) für die aus dem Reaktor stammende Gasphase sowie einem Kreisgasverdichter, der die Gasphase aus dem Phasentrennbehälter wieder unten in die Reaktionszone zurück führt. Ein Teil dieses Kreisgases wird nach der Phasentrennung als Abgas aus dem Reaktionssystem gefahren. Um eine optimale Gasverteilung im Reaktorsystem zu realisieren, wurde hier ein Gasverteilerring mit Bohrungen verbaut. Über installierte Heiz- und Kühlvorrichtungen konnte der Reaktor temperiert werden.

Vor der Hydroformylierung wurde das System mit Stickstoff frei von Sauerstoff gespült. Anschließend wurde der Reaktor mit 12 Liter Katalysatorlösung gefüllt.

Diese Katalysatorlösung setzte sich aus 12 kg eines eutektischen Gemisches aus Biphenyl und Diphenylether (Diphyl^{®} ,Wärmeträgeröl der Fa. Lanxess), 3 g Rh(acac)(CO)₂, 36 g Bisphosphit-Ligand der Formel (4), 67.5 g Amin der Formel (5) zusammen und wurde vorher in einem Behälter gemischt. Das eutektische Gemisch aus Biphenyl und Diphenylether (Diphyl^{®}) wurde zuvor mit Stickstoff gestrippt, um Sauerstoff und Wasser aus dem Wärmeträgeröl zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich um eine Mischung aus 35 Gew.-% 2-Butenen und 1-Buten in einer Konzentration von ca. 1 %. Der Rest war n-Butan.

Folgende Durchsätze wurden eingestellt: 0,3 kg/h Einsatzgemisch, 75 NI/h Synthesegas (50 Vol% H₂ und 50 Vol% CO)

Zur täglichen Dosierung des Bisphosphit-Liganden (4) und Amins (5) wurde eine 1.4%-ige Lösung des Bisphosphit-Liganden (4) in n-Pentanal angesetzt, welches zuvor durch Strippen mit Stickstoff von restlichen C₄-Kohlenwasserstoffen (< 3 %) befreit wurde. Das Amin (5) wurde in einem dreifachen molaren Überschuss zum Bisphosphit-Liganden (4) eingesetzt. Zur besseren Stabilisierung dieser Lösung wurde das Amin (5) vor dem Bisphosphit-Liganden (4) zur Lösung gegeben.

Nach ca. 1000 h wurde ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.

Durch eine tägliche Dosierung der oben beschriebenen Ligandenlösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht. Unter den gewählten Reaktionsbedingungen wurden Butenumsätze von rund 65 bis 70 % erzielt. Die prozentuale Verteilung zwischen n-Pentanal und 2-Methylbutanal, bzw. die n/iso-Selektivität, betrug 95 % zu 5 %.In der stationären Phase des Versuches konnte kein Rhodiumabbau verzeichnet werden.

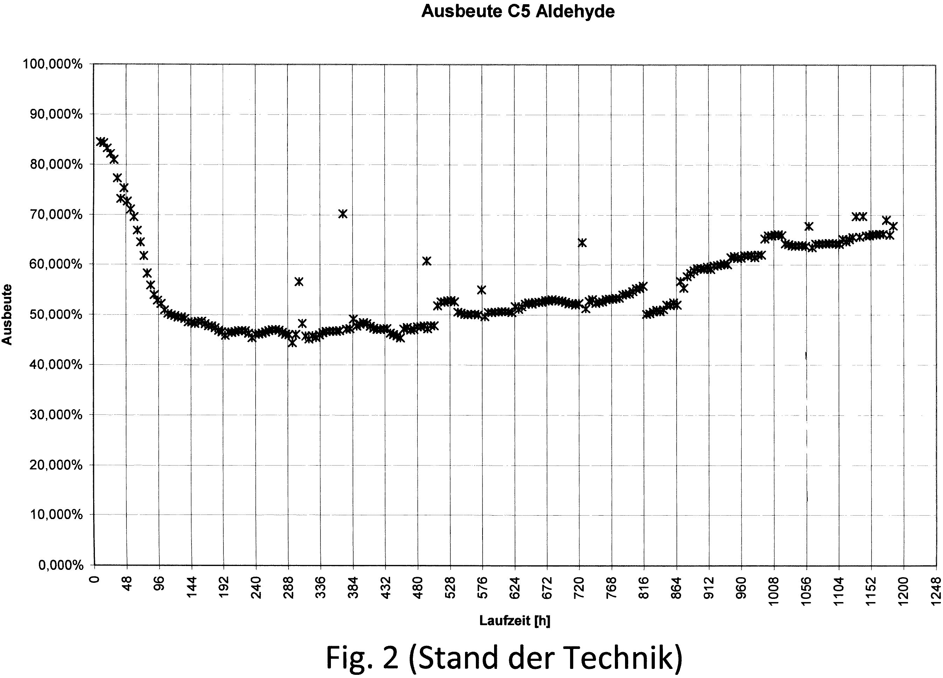

Die Ausbeute der C₅-Aldehyde über die Versuchszeit ist in Figur 2 aufgetragen.
- Figur 2:: Pentanal-Ausbeute zu Beispiel 1

Nach 1200 h wurde der Reaktor entspannt und die Katalysatorlösung untersucht. Im Reaktor zeigte sich ein Niederschlag. Eine Analyse dieses Niederschlages ergab, dass dieser aus phosphorhaltigen Folgeprodukten des Bisphosphit-Liganden (4) und dem eingesetzten Amin (5) bestanden. Es wurden keinerlei Anbackungen dieser Ausfällungen in dem Reaktor festgestellt.

Ein Teil des Reaktorinhaltes wurde, nach Abtrennung des Niederschlages, bei 1.2 KPa abs. und 220 °C Sumpftemperatur auf 13 % bezogen auf die Einsatzmasse eingeengt. Der erhaltene Rückstand aus dem Sumpf war noch fließfähig und es wurde kein Niederschlag festgestellt. Eine Rhodiumanalyse zeigte, dass sich das gesamte Rhodium aus der Einsatzmasse in diesem Sumpfrückstand befand.

### Beispiel 2: Hydroformylierung mit dem nicht erfindungsgemäßen Liganden (4) über 8000 h

Die Versuchsdurchführung fand in der, in Beispiel 1 beschriebenen Versuchsanlage statt. Die Vorbereitung des Versuches und die Durchführung fand analog zum Bespiel 1 statt.

In diesem Beispiel setzte sich die Katalysatorlösung aus 12 kg Isononylbenzoat, 4.5 g Rh(acac)(CO)₂, 55 g Bisphosphit-Ligand der Formel (4), 67.5 g Amin der Formel (5) zusammen. Das Isononylbenzoat wurde ebenfalls zuvor mit Stickstoff gestrippt, um Sauerstoff und Wasser aus dem Lösemittel zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Anschließend wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich um eine Mischung aus 35 Gew.-% 2-Butenen und 1-Buten in einer Konzentration von ca. 1 %. Der Rest war n-Butan. Folgende Durchsätze wurden eingestellt: 0,3 kg/h Einsatzgemisch, 75 NI/h Synthesegas (50 Vol% H₂ und 50 Vol% CO).

Zur täglichen Dosierung des Bisphosphit-Liganden (4) und Amins (5) wurde eine 1.4%-ige Lösung des Bisphosphit-Liganden (4) in n-Pentanal angesetzt, welches zuvor durch Strippen mit Stickstoff von restlichen C₄-Kohlenwasserstoffen (< 3 %) befreit wurde. Das Amin (5) wurde in einem dreifachen molaren Überschuss zum Bisphosphit-Liganden (4) eingesetzt. Zur besseren Stabilisierung dieser Lösung wurde das Amin (5) vor dem Bisphosphit-Liganden (4) zur Lösung gegeben.

Wie in Beispiel 1 wurde nach etwa 1000 h ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.

Durch eine tägliche Dosierung der oben beschriebenen Ligandenlösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht. Unter den gewählten Reaktionsbedingungen wurden Butenumsätze von rund 65 bis 70 % erzielt. Die prozentuale Verteilung zwischen n-Pentanal und 2-Methylbutanal, bzw. die n/iso-Selektivität, betrug 95 % zu 5 %.In der stationären Phase des Versuches konnte kein Rhodiumabbau verzeichnet werden.

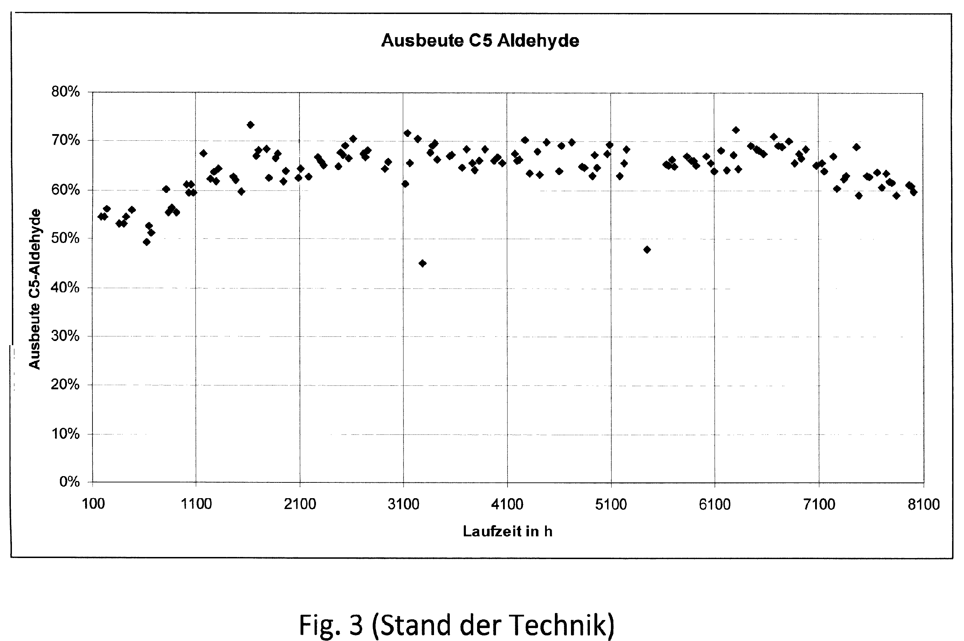

Die Ausbeute der C₅-Aldehyde über die Versuchszeit ist in Figur 3 aufgetragen.
- Figur 3:: Pentanal-Ausbeute zu Beispiel 2

Nach 1500 h zeigten sich in den Proben aus dem Reaktor erste Niederschläge. Die Analyse dieser Niederschläge ergab, dass dieser, ebenso wie in Beispiel 1, aus phosphorhaltigen Folgeprodukten des Bisphosphit-Liganden (4) und dem eingesetzten Amin (5)bestanden.

Die Reaktion wurde insgesamt 8100 h betrieben, die Rhodiumverluste durch Probenahmen wurden durch Zugabe entsprechender Mengen Rh(acac)(CO)₂ in die tägliche Ligandendosierungslösung ausgeglichen.

Im Verlauf wurde nach ca. 7000 h ein Aktivitätsrückgang in der Reaktion beobachtet und die Reaktionslösung neigte zum Schäumen. Der Prozess konnte nicht mehr betrieben werden und der Versuch musste beendet werden.

Nach Ende der Reaktion wurde der Reaktor entspannt und die Reaktionsmischung untersucht. Es zeigten sich große Mengen an Feststoff. 250 ml der Reaktionslösung wurden 4 h unter N₂ Atmosphäre bei 40°C gerührt und anschließend die Viskosität des Rückstands vermessen. Die Viskosität betrug 300 mPas.

### Beispiel 3: Herstellung erfindungsgemäß eingesetzter Liganden (1) und (2)

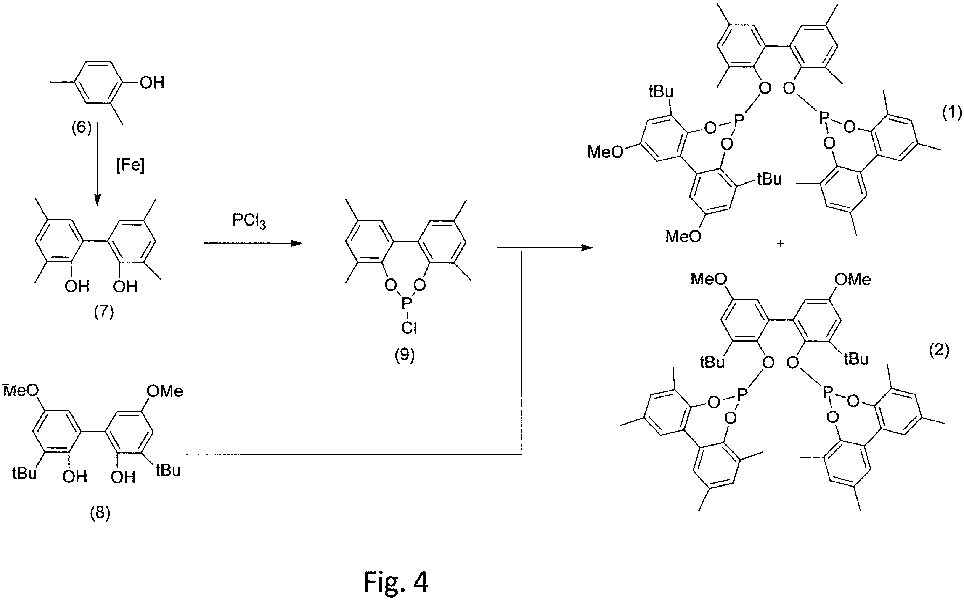

Die allgemeine Reaktionsgleichung zur Herstellung eines erfindungsgemäß eingesetzten Katalysatorsystems ist in Figur 4 dargestellt.
- Figur 4:: Herstellung erfindungsgemäßer Liganden

Im Folgenden werden diese Abkürzungen verwendet:
VE-Wasser = demineralisiertes Wasser
KPG = Kerngezogenes Präzisions-Glasgerät
ACN = Acetonitril
EtOAc = Ethylacetat
DMAB = Dimethylaminobutan
NMP = N-Methylpyrrolidon
ÖV = Ölvakuum
acac = acetylacetonat
NEt₃ = Triethylamin
TIPB = 1,2,4,5-Tetraisopropylbenzol

### Synthese des 2,2'-Bis(3,5-dimethylphenol) nach Formel (7):

Das als Vorstufe eingesetzte Biphenol (7) wurde nach folgender Synthesevorschrift hergestellt.

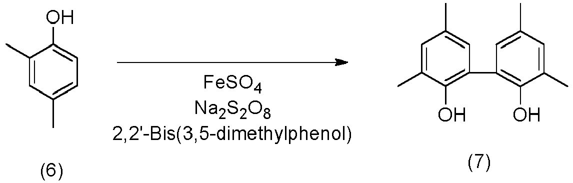

In einem 500 ml Schlenk mit KPG-Rührer, Zwischenaufsatz und Glasrührer wurden 1,42 g (0,005 mol) Eisen(II)-sulfatheptahydrat und 12,35 g (0,1 mol) 2,4-Dimethylphenol (6) in 150 ml VE-Wasser und 5 ml Cyclohexan vorgelegt und auf 40 °C erwärmt.

In einem 100 ml Becherglas löste man 25.36 g (0,146 mol) Natriumperoxodisulfat in 80 ml VE-Wasser. Zum Start der Reaktion wurde eine kleine Portion Na₂S₂O₈-Lösung zum Phenol gegeben. Anschließend wurde alle 10 min eine kleinere Portion der Lösung hinzugegeben. Nach 30 min war die Na₂S₂O₈-Lösung hinzugegeben.

Nach einer Reaktionszeit von 5h wurde zur Reaktionslösung 300 ml Cyclohexan und 200 ml Wasser hinzugegeben, 20 min rühren gelassen, dann warm in den Scheidetrichter überführt.

Die organische Phase wurde abgetrennt und bis zur Trockene eingeengt. Das Produkt konnte in 69%iger Ausbeute (10,6g) erhalten werden.

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Die Charakterisierung des Produktes erfolgte mittels NMR-Spektroskopie (Bruker Avance 500 MHz FT-NMR-Spektrometer). Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795 - 1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde das Mengenverhältnis der beiden Liganden (Ligand (1) und Ligand (2)) zueinander bestimmt. Der unsymmetrische Ligand (1) wird durch zwei Phosphorsignale im Bereich von (δ) = 140,6 ppm bis (δ) = 142,8 ppm charakterisiert, wohingegen für der symmetrischen Ligand (2) nur ein Phosphorsignal im Bereich (δ) = 139, 1 ppm bis (δ) = 139,8 ppm aufweist.

### Synthese des 2,2'-Bis-(3,5-dimethylphenol)chlorophosphits (9):

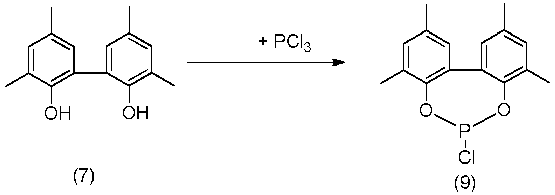

In einem sekurierten 2 L Schlenk mit Magnetrührer wurden 440 ml (692.56 g) Phosphortrichlorid vorgelegt. In einem zweiten sekurierten 1 L Schlenk wurden 120 g 2,2'-Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrichlorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCl-Verbindungen, 0,8% P-H-Verbindung.

### Herstellung des Isomerengemisches, bestehend aus den Liganden (1) und (2):

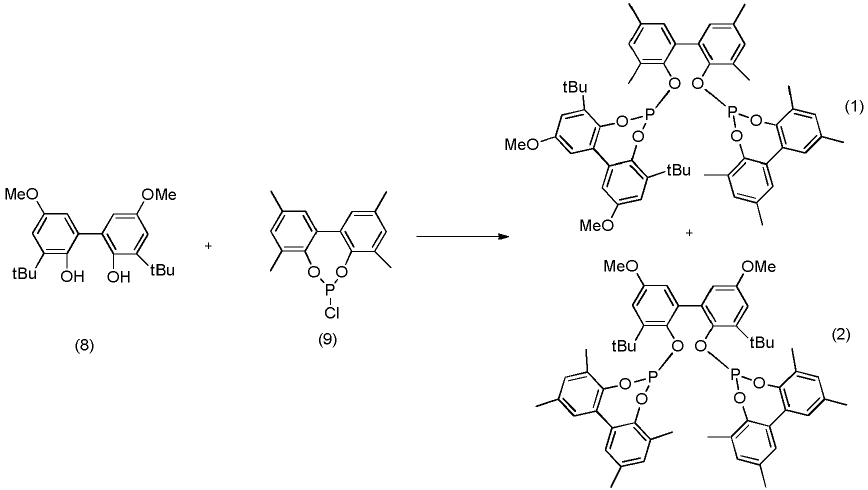

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem Triethylamin (0,29 mol) versetzt. Dann wurde langsam die Biphenol/Trietylamin-Lösung zu der Chlorophosphitlösung getropft. Nach einer Nachreaktionszeit von 1 h wurde die Reaktionslösung über Nacht bei 45°C gerührt.

Anschließend wurde die Lösung filtriert und der Feststoff dreimal mit 100 ml warmen (45°C) ACN gewaschen. Das Zielprodukt konnte als weißer Feststoff (43,3g, 86%) erhalten werden. 31 P-NMR (202,4 MHz, toluene-d8): 142,5 und 140,9 (95,4%) 139,2 (4,6%).

### Beispiel 4: Hydroformylierung mit erfindungsgemäßem Katalysatorsystem

Es wurde dieselbe Versuchsanlage eingesetzt wie in Beispiel 1. Es wurde dasselbe Einsatzgemisch und dasselbe Synthesegas verwendet. Als Ligand wurde indes eine Mischung aus den beiden Bisphosphit-Liganden (1) und (2) eingesetzt, die gemäß Beispiel 3 hergestellt wurde. Der aus EP2280920B1 bekannte Ligand der Formel (4) war nicht im Reaktionsgemisch enthalten. Es wurde dasselbe Amin (5) wie im Vergleichsbeispiel 1 als Stabilisator verwendet. Als Lösungsmittel wurde Isononylbenzoat eingesetzt.

Vor der Hydroformylierung wurde das System mit Stickstoff frei von Sauerstoff gespült. Anschließend wurde der Reaktor mit 12 Liter Katalysatorlösung gefüllt.

Diese Katalysatorlösung setzte sich aus 12 kg Isononylbenzoat, 4.5 g Rh(acac)(CO)2, 63 g Liganden-Isomerengemisch der Formeln (1) und (2), 200g Amin der Formel (5) zusammen und wurde vorher in einem Behälter gemischt. Das Isononylbenzoat wurde zuvor mit Stickstoff gestrippt, um Sauerstoff und Wasser aus dem Lösemittel zu entfernen.

Anschließend wurde das Reaktorsystem mit Synthesegas frei von Stickstoff gespült. Nachdem der Stickstoffgehalt unter 10 Vol-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Das Einsatzgemisch wurde über einen Verdampfer gefahren, um es gasförmig in das Kreisgas zu fahren. Folgende Durchsätze wurden eingestellt: 0.3 kg/h Einsatzgemisch, 75 NI/h Synthesegas.

Zur täglichen Dosierung des Isomerengemisches bestehend aus (1) und (2) und Amins (5) wurde eine 1.4%-ige Lösung der Ligandenmischungen aus den Bisphosphit-Liganden (1) und (2) in n-Pentanal angesetzt, welches zuvor durch Strippen mit Stickstoff von restlichen C₄-Kohlenwasserstoffen (< 3 %) befreit wurde. Das Amin (5) wurde in einem dreifachen molaren Überschuss zum Ligandenisomerengemisch bestehend aus (1) und (2) eingesetzt. Zur besseren Stabilisierung dieser Lösung wurde das Amin (5) vor dem Bisphosphit-Ligandenisomerengemisch zur Lösung gegeben.

Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Ausbeutebestimmung wurden aus dem Kreisgas vor und nach Reaktor Proben gezogen und mittels Gaschromatograph analysiert.

Durch eine tägliche Dosierung der oben beschriebenen Ligandenlösung, konnte der Umsatz und die Regioselektivität konstant gehalten werden. Zur Bestimmung des Reaktorinhaltes wurden Proben aus dem Reaktor entnommen und mittels Flüssigchromatographie (HLPC) untersucht.

Unter den gewählten Reaktionsbedingungen stellte sich zum Start der Reaktion eine Aldehydausbeute zwischen 80% und 90% ein. Nach 8000 h Betriebszeit fiel die Ausbeute auf ca. 65% ab, bedingt durch die Rhodiumverluste durch die Probennahmen. Ein Schäumen der Reaktionslösung konnte in diesem Fall nicht festgestellt werden. Die prozentuale Verteilung zwischen n-Pentanal und 2-Methylbutanal, bzw. die Regio-Selektivität, betrug 92 % zu 8 %.

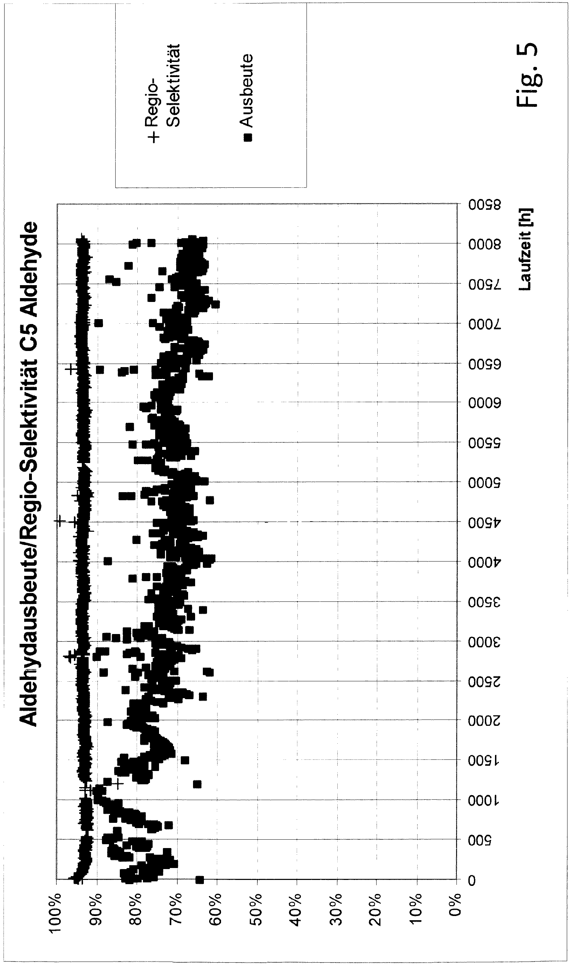

Aldehydausbeute und Regio-Selektivität sind über die Versuchsdauer in Figur 5 aufgetragen.
- Figur 5:: Aldehydausbeute und Regio-Selektivität zu Beispiel 4

In der stationären Phase des Versuches konnte, abgesehen von den Rhodiumverlusten durch die Probenahme, kein weiterer Rhodiumabbau verzeichnet werden.

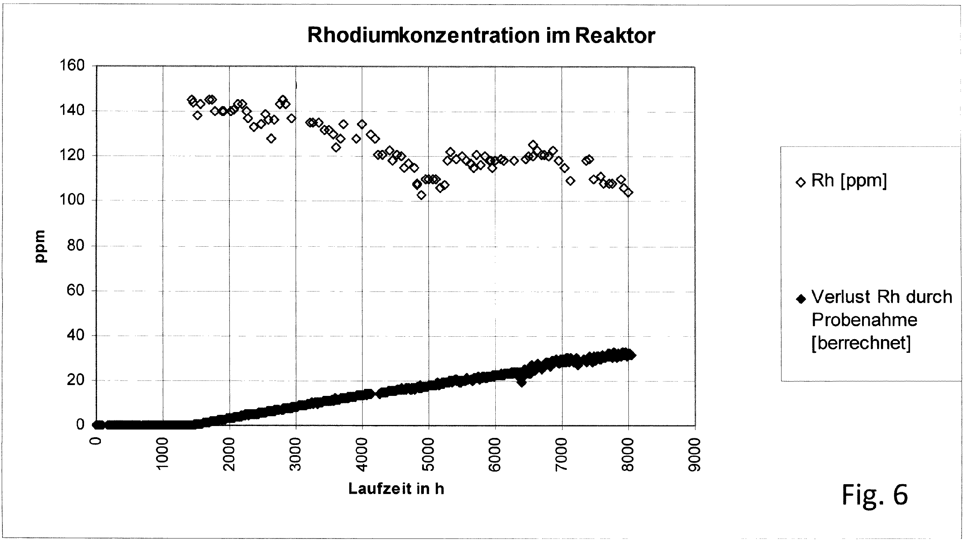

Die Rhodium-Konzentration im Reaktor über die Versuchsdauer ist in Figur 6 aufgetragen.
- Figur 6:: Rh-Konzentration zu Beispiel 4

Nach Ende der Reaktion wurde der Reaktor entspannt und die Reaktionsmischung untersucht. Es zeigte sich kein Feststoff. 250ml der Reaktionslösung wurden 4 h unter N₂ Atmosphäre bei 40 °C gerührt und anschließend die Viskosität des Rückstands vermessen. Die Viskosität betrug 20 mPas.

### Vergleich der Beispiele 1,2 und 4

Vergleicht man die entsprechenden Beispiele, so hebt sich das erfindungsgemäß durchgeführte Beispiel 4 durch folgende Merkmale deutlich von den Beispielen 1 und 2 ab, die den Stand der Technik wiedergegeben:
Das erfindungsgemäße Beispiel 4 zeigt keine Einfahrphase, das heißt das System zeigt keinen Aktivitätsrückgang in den ersten 1000 h Betriebszeit und somit produziert die Anlage im erfindungsgemäßen Beispiel im gleichen Zeitraum deutlich mehr Produkt.

Im Vergleichsbeispiel 2 fällt im Laufe der Reaktion Feststoff an, der nur über eine aufwendige Filtration entfernt werden kann. Das erfindungsgemäße Beispiel 4 zeigt auch nach über 8000 h keinen Feststoffanfall, somit kann in diesem Verfahren auf die Filtration verzichtet werden.

Das Vergleichsbeispiel 2 zeigt ein deutliches Schäumen der Reaktionslösung zum Ende des Versuches, sodass der Prozess nicht mehr betrieben werden kann. Ein solches Verhalten ließe sich nur durch aufwendige Schaumbrecher verhindern. Das erfindungsgemäße Verfahren kommt ohne diese Hilfsmittel aus.

## Patentansprüche

1. Verfahren zur Herstellung von Aldehyden mit fünf Kohlenstoffatomen, bei welchem ein 10 Gew.-% bis 50 Gew.-% lineare Butene und weniger als 5 Gew.-% 1-Buten enthaltendes Einsatzgemisch mit Synthesegas in Gegenwart eines Rhodium sowie mindestens einen Bisphosphit-Liganden umfassenden Katalysatorsystems hydroformyliert wird, wobei die Hydroformylierung in einem Reaktor erfolgt, aus welchem über einen Betriebszeitraum kontinuierlich ein Kreisgas enthaltend zumindest ein Teil der Produkte sowie nicht umgesetzte Edukte der Hydroformylierung abgezogen, teilkondensiert und nicht kondensierte Anteile des Kreisgases in den Reaktor zurückgeführt wird, und wobei nach Ablauf des Betriebszeitraums die Hydroformylierung unterbrochen, der Reaktor von Reaktionsrückständen befreit und die Hydroformylierung wieder aufgenommen wird,
**dadurch gekennzeichnet,**
**dass** der Betriebszeitraum mindestens 8000 h andauert,
**dass** während des andauernden Betriebszeitraums auf eine Abscheidung fester Reaktionsrückstände aus dem Reaktor verzichtet wird,
und **dass** das Katalysatorsystem den Bisphosphit-Liganden der Formel (1) und/oder den Bisphosphit-Liganden der Formel (2) umfasst:

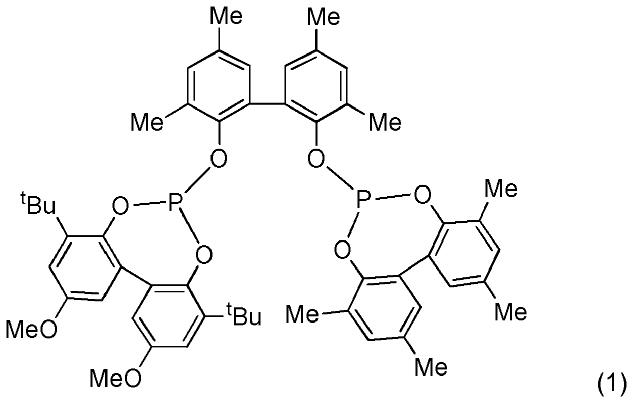

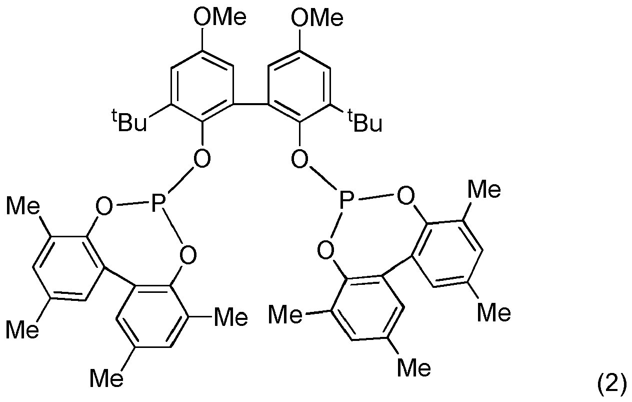

2. Verfahren nach Anspruch 1,
**dadurch gekennzeichnet,**
**dass** das Katalysatorsystem sowohl den Bisphosphit-Liganden der Formel (1), als auch den Bisphosphit-Liganden der Formel (2) umfasst.

3. Verfahren nach Anspruch 2,
**dadurch gekennzeichnet,**
**dass** in der Hydroformylierung das molare Verhältnis der Bisphosphit-Liganden der Formel (1) zu den Bisphosphit-Liganden der Formel (2) zwischen 10 und 30 liegt.

4. Verfahren nach einem der Ansprüche 1 bis 3,
**dadurch gekennzeichnet,**
**dass** die Hydroformylierung in Gegenwart eines organischen Amins der Formel (3) durchgeführt wird, 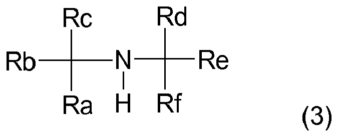 worin Ra, Rb, Rc, Rd, Re und Rf gleiche oder unterschiedliche Kohlenwasserstoffreste darstellen, die auch untereinander verbunden sein können.

5. Verfahren nach Anspruch 4,
**dadurch gekennzeichnet,**
**dass** das organische Amin zumindest eine 2,2,6,6-Tetramethylpiperidineinheit aufweist.

6. Verfahren nach Anspruch 5,
**dadurch gekennzeichnet, dass** das organische Amin ein Sebacinsäuredi-4-(2,2,6,6-tetramethylpiperidinyl)-ester ist.

7. Verfahren nach einem der Ansprüche 1 bis 6,
**dadurch gekennzeichnet,**
**dass** die Hydroformylierung in Gegenwart von Isononylbenzoat durchgeführt wird.

8. Verfahren nach einem der Ansprüche 1 bis 7,
**dadurch gekennzeichnet,**
**dass** das Kreisgas bei einer Kondensationstemperatur von 50°C bis 90°C teilkondensiert wird; bevorzugt, dass die Kondensationstemperatur zwischen 65°C und 75°C liegt; ganz besonders bevorzugt, dass sie bei 70°C liegt.

9. Verfahren nach einem der Ansprüche 1 bis 8,
**dadurch gekennzeichnet,**
**dass** der Betriebszeitraum 12000 h oder mehr beträgt.

10. Verfahren nach einem der Ansprüche 1 bis 9,
**dadurch gekennzeichnet,**
**dass** das Einsatzgemisch 10 bis 50 Gew.-% lineare Butene, weniger als 2 Gew.-% 1-Buten und mindestens 50 Gew.-% Butane enthält.

11. Verfahren nach einem der Ansprüche 1 bis 10,
**dadurch gekennzeichnet,**
**dass** die Hydroformylierung bei den folgenden Reaktionsbedingungen durchgeführt wird:
| | |
|---|---|
| Druck: | 1 MPa bis 20 MPa; |
| Temperatur: | 70°C bis 150°C; |
| Rhodiumkonzentration: | 1 Gew.-ppm bis 1000 Gew.-ppm; |
| Ligand/Rhodium-Verhältnis: | 1 bis 100. |

12. Anlage für die Durchführung eines Verfahrens nach einem der Ansprüche 1 bis 11, umfassend einen Reaktor zur Herstellung von Aldehyden mit fünf Kohlenstoffatomen durch Hydroformylierung von linearen Butenen und Synthesegas in Gegenwart eines Rhodium sowie mindestens einen Bisphosphit-Liganden umfassenden Katalysatorsystems, weiter umfassend Mittel zum Abzug eines Kreisgases enthaltend zumindest ein Teil der Produkte sowie nicht umgesetzte Edukte der Hydroformylierung aus dem Reaktor, weiter umfassend Mittel zum Teilkondensieren des Kreisgases und zum Rückführen der nicht kondensierten Anteile des Kreisgases in den Reaktor,
**dadurch gekennzeichnet,**
**dass** der Reaktor den Bisphosphit-Liganden der Formel (1) und/oder den Bisphosphit-Liganden der Formel (2) enthält.

## Claims

1. Process for preparing aldehydes having five carbon atoms, in which an input mixture containing 10% by weight to 50% by weight of linear butenes and less than 5% by weight of 1-butene is hydroformylated with synthesis gas in the presence of a catalyst system comprising rhodium and at least one bisphosphite ligand, wherein the hydroformylation is effected in a reactor from which, over an operating period, a cycle gas containing at least a portion of the products and unconverted reactants from the hydroformylation is continuously drawn off and partly condensed, and uncondensed components of the cycle gas are recycled into the reactor, and wherein, after the operating period has expired, the hydroformylation is stopped, the reactor is freed of reaction residues and the hydroformylation is restarted,
**characterized in that**
the operating period lasts for at least 8000 h,
there is no separation of solid reaction residues out of the reactor during the course of the operating period,
and **in that** the catalyst system comprises the bisphosphite ligand of the formula (1) and/or the bisphosphite ligand of the formula (2):

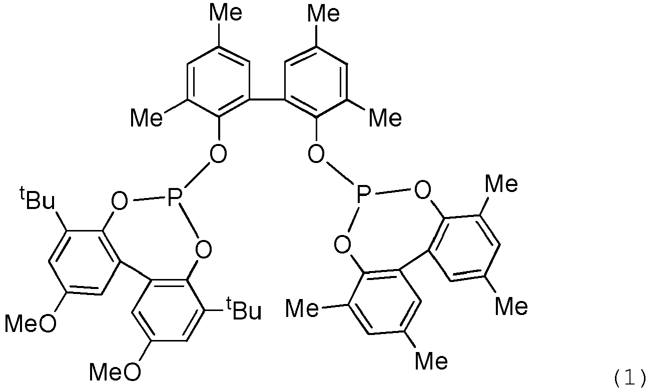

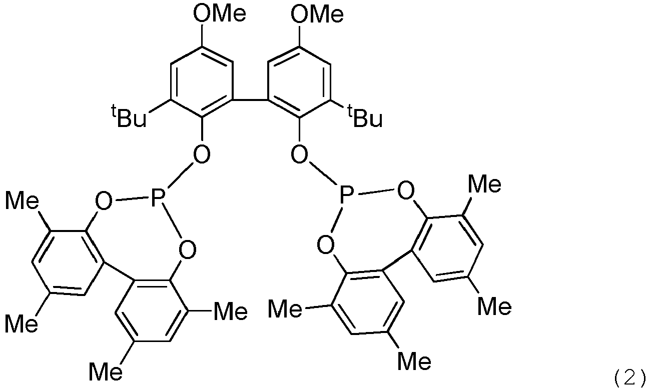

2. Process according to Claim 1,
**characterized in that**
the catalyst system comprises both the bisphosphite ligand of the formula (1) and the bisphosphite ligand of the formula (2).

3. Process according to Claim 2,
**characterized in that**
the molar ratio of the bisphosphite ligands of the formula (1) to the bisphosphite ligands of the formula (2) in the hydroformylation is between 10 and 30.

4. Process according to any of Claims 1 to 3,
**characterized in that**
the hydroformylation is conducted in the presence of an organic amine of the formula (3) 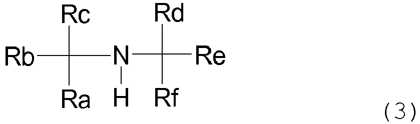 in which Ra, Rb, Rc, Rd, Re and Rf are identical or different hydrocarbyl radicals which may also be joined to one another.

5. Process according to Claim 4,
**characterized in that**
the organic amine has at least one 2,2,6,6-tetramethylpiperidine unit.

6. Process according to Claim 5,
**characterized in that** the organic amine is a di-4-(2,2,6,6-tetramethylpiperidinyl) sebacate.

7. Process according to any of Claims 1 to 6,
**characterized in that**
the hydroformylation is conducted in the presence of isononyl benzoate.

8. Process according to any of Claims 1 to 7,
**characterized in that**
the cycle gas is partly condensed at a condensation temperature of 50°C to 90°C, the condensation temperature preferably being between 65°C and 75°C, most preferably 70°C.

9. Process according to any of Claims 1 to 8,
**characterized in that**
the operating period is 12 000 h or more.

10. Process according to any of Claims 1 to 9,
**characterized in that**
the input mixture contains 10% to 50% by weight of linear butenes, less than 2% by weight of 1-butene and at least 50% by weight of butanes.

11. Process according to any of Claims 1 to 10,
**characterized in that**
the hydroformylation is conducted under the following reaction conditions:
| | |
|---|---|
| Pressure: | 1 MPa to 20 MPa; |
| Temperature: | 70°C to 150°C; |
| Rhodium concentration: | 1 ppm by weight to 1000 ppm by weight; |
| Ligand/rhodium ratio: | 1 to 100. |

12. Plant for the performance of a process according to any of Claims 1 to 11, comprising a reactor for preparation of aldehydes having five carbon atoms by hydroformylation of linear butenes and synthesis gas in the presence of a catalyst system comprising rhodium and at least one bisphosphite ligand, further comprising means for drawing off a cycle gas containing at least a portion of the products and unconverted reactants from the hydroformylation from the reactor, further comprising means for partial condensation of the cycle gas and for recycling of the uncondensed components of the cycle gas into the reactor,
**characterized in that**
the reactor contains the bisphosphite ligand of the formula (1) and/or the bisphosphite ligand of the formula (2).

## Revendications

1. Procédé de préparation d'aldéhydes contenant cinq atomes de carbone, selon lequel un mélange de départ contenant 10 % en poids à 50 % en poids de butènes linéaires et moins de 5 % en poids de 1-butène est hydroformylé avec un gaz de synthèse en présence d'un système catalytique comprenant du rhodium et au moins un ligand bisphosphite, l'hydroformylation ayant lieu dans un réacteur duquel un gaz circulaire contenant au moins une partie des produits, ainsi que des réactifs non réagis de l'hydroformylation, est soutiré en continu pendant une durée d'exploitation, partiellement condensé et les fractions non condensées du gaz circulaire sont recyclées dans le réacteur, et l'hydroformylation étant interrompue après l'écoulement de la durée d'exploitation, le réacteur étant débarrassé des résidus de réaction et l'hydroformylation étant redémarrée,
**caractérisé en ce que**
la durée d'exploitation est d'au moins 8 000 h,
un dépôt de résidus de réaction solides du réacteur pendant la durée d'exploitation est supprimé,
et le système catalytique comprend le ligand bisphosphite de formule (1) et/ou le ligand bisphosphite de formule (2) :

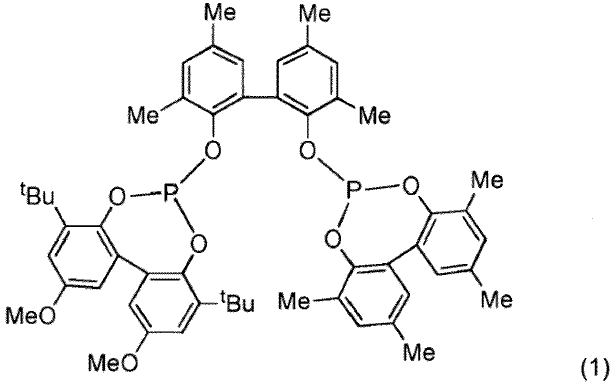

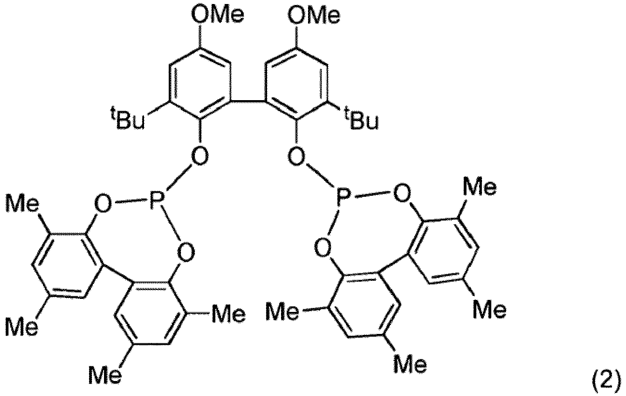

2. Procédé selon la revendication 1, **caractérisé en ce que** le système catalytique comprend aussi bien le ligand bisphosphite de formule (1) que le ligand bisphosphite de formule (2).

3. Procédé selon la revendication 2, **caractérisé en ce que**, lors de l'hydroformylation, le rapport molaire entre le ligand bisphosphite de formule (1) et le ligand bisphosphite de formule (2) est compris entre 10 et 30.

4. Procédé selon l'une quelconque des revendications 1 à 3, **caractérisé en ce que** l'hydroformylation est réalisée en présence d'une amine organique de formule (3) 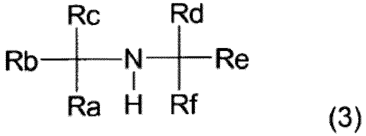 dans laquelle Ra, Rb, Rc, Rd, Re et Rf représentent des radicaux hydrocarbonés identiques ou différents, qui peuvent également être reliés entre eux.

5. Procédé selon la revendication 4, **caractérisé en ce que** l'amine organique comprend au moins une unité 2,2,6,6-tétraméthylpipéridine.

6. Procédé selon la revendication 5, **caractérisé en ce que** l'amine organique est l'ester di-4-(2,2,6,6-tétraméthylpipéridinylique) de l'acide sébacique.

7. Procédé selon l'une quelconque des revendications 1 à 6, **caractérisé en ce que** l'hydroformylation est réalisée en présence de benzoate d'isononyle.

8. Procédé selon l'une quelconque des revendications 1 à 7, **caractérisé en ce que** le gaz circulaire est partiellement condensé à une température de condensation de 50 °C à 90 °C ; de préférence **en ce que** la température de condensation est comprise entre 65 °C et 75 °C ; de manière particulièrement préférée en ce qu'elle est de 70 °C.

9. Procédé selon l'une quelconque des revendications 1 à 8, **caractérisé en ce que** la durée d'exploitation est de 12 000 h ou plus.

10. Procédé selon l'une quelconque des revendications 1 à 9, **caractérisé en ce que** le mélange de départ contient 10 à 50 % en poids de butènes linéaires, moins de 2 % en poids de 1-butène et au moins 50 % en poids de butanes.

11. Procédé selon l'une quelconque des revendications 1 à 10, **caractérisé en ce que** l'hydroformylation est réalisée dans les conditions de réaction suivantes :
| | |
|---|---|
| pression : | 1 MPa à 20 MPa ; |
| température : | 70 °C à 150 °C ; |
| concentration de rhodium : en poids ; | 1 ppm en poids à 1 000 ppm |
| rapport ligand/rhodium : | 1 à 100. |

12. Unité pour la réalisation d'un procédé selon l'une quelconque des revendications 1 à 11, comprenant un réacteur pour la préparation d'aldéhydes contenant cinq atomes de carbone par hydroformylation de butènes linéaires et d'un gaz de synthèse en présence d'un système catalytique comprenant du rhodium et au moins un ligand bisphosphite, comprenant en outre un moyen de soutirage d'un gaz circulaire contenant au moins une partie des produits, ainsi que des réactifs non réagis de l'hydroformylation, du réacteur, comprenant en outre un moyen de condensation partielle du gaz circulaire et de recyclage des fractions non condensées du gaz circulaire dans le réacteur,
**caractérisée en ce que** le réacteur contient le ligand bisphosphite de formule (1) et/ou le ligand bisphosphite de formule (2).
